(19) Europäisches Patentamt
European Patent Office
Office européen des brevets

(11) Publication number: 0 193 660 A1

(12) # EUROPEAN PATENT APPLICATION

(21) Application number: 85301443.9

(22) Date of filing: 01.03.85

(51) Int. Cl.⁴: C 07 D 249/12, A 01 N 43/64

(43) Date of publication of application: 10.09.86 Bulletin 86/37

(84) Designated Contracting States: BE CH DE FR GB IT LI NL

(71) Applicant: NIHON NOHYAKU CO., LTD., 1-2-5, Nihonbashi, Chuo-ku Tokyo (JP)

(72) Inventor: Matsui, Hisanori, 12-24 Honcho-4-chome, Toda-shi (JP)
Inventor: Sutoh, Keiji, 7-20, Nikawayurinocho, Nishinomiya-shi (JP)
Inventor: Yamamoto, Moriharu, 11-1, Nishiokamoto-6-chome, Higashinada Kobe (JP)

(74) Representative: Grundy, Derek George Ritchie, CARPMAELS & RANSFORD 43, Bloomsbury Square, London WC1A 2RA (GB)

(54) 1-(Hydroxyphenyl)-3-methyl-4-difluoromethyl-(delta-2)-1,2,4-triazolin-5-one derivatives.

(57) Novel triazolinone derivatives represented by the general formula (I)

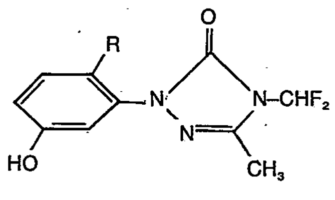

[wherein R is a hydrogen atom or a fluorine atom].
The triazolinone derivatives are useful as intermediates for preparing agricultural chemicals, particularly for preparing useful herbicides.

EP 0 193 660 A1

# NOVEL 1-(HYDROXYPHENYL)-3-METHYL-4-DIFLUOROMETHYL-$\Delta^2$-1,2,4-TRIAZOLIN-5-ONE DERIVATIVES

## FIELD OF THE INVENTION

The present invention relates to novel triazolinone derivatives and process for preparing the same.

The triazolinone derivatives of the present invention are represented by the general formula (I),

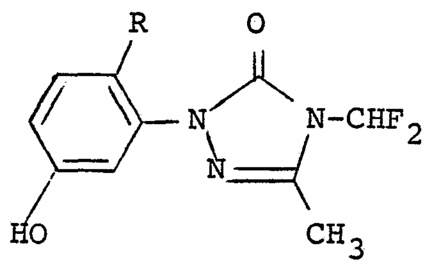

(I)

[wherein R is a hydrogen atom or a fluorine atom].

The triazolinone derivatives of the present invention have not been known yet from any of literature, and thus they are novel compounds. They are useful as intermediates for preparing agricultural chemicals, particularly for preparing useful herbicides.

## DESCRIPTION OF THE PRIOR ART

Some of herbicides described in U. S. Patent No. 4,398,943 may be derived from triazolinone derivatives of the present invention as the intermediates.

## SUMMARY OF THE INVENTION

An object of the present invention is to provide novel triazolinone derivatives.

Another object of the present invention is to provide process for preparing the triazolinone derivatives.

## DETAILED DESCRIPTION OF PREFERRED EMBODIMENTS

The triazolinone derivatives of the present invention represented by the general formula (I),

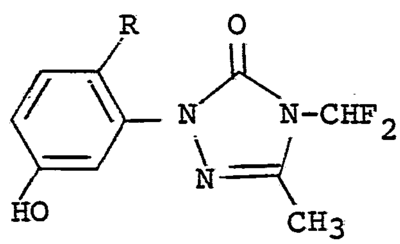

(I)

[wherein R is the same as defined above], may be prepared by diazotizing 1-(aminophenyl)-3-methyl-4-difluoromethyl-$\Delta^2$-1,2,4-triazolin-5-one represented by the general formula (II),

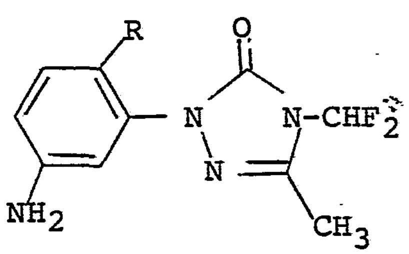

(II)

[wherein R is the same as defined above], with sodium nitrite in an aqueous sulfuric acid by a method commonly used in diazotization, to obtain the

corresponding diazotized product represented by the general formula (II'),

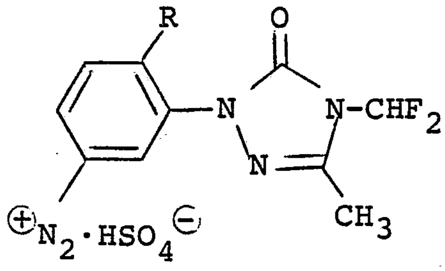

(II')

[wherein R is the same as defined above], then the resulting diazotized product, without being separated from the reaction mixture is thermally decomposed to yield the desired 1-(hydroxyphenyl)-3-methyl-4-difluoromethyl-$\Delta^2$-1,2,4-triazolin-5-one represented by the general formula (I). These reaction are shown in the following reaction scheme.

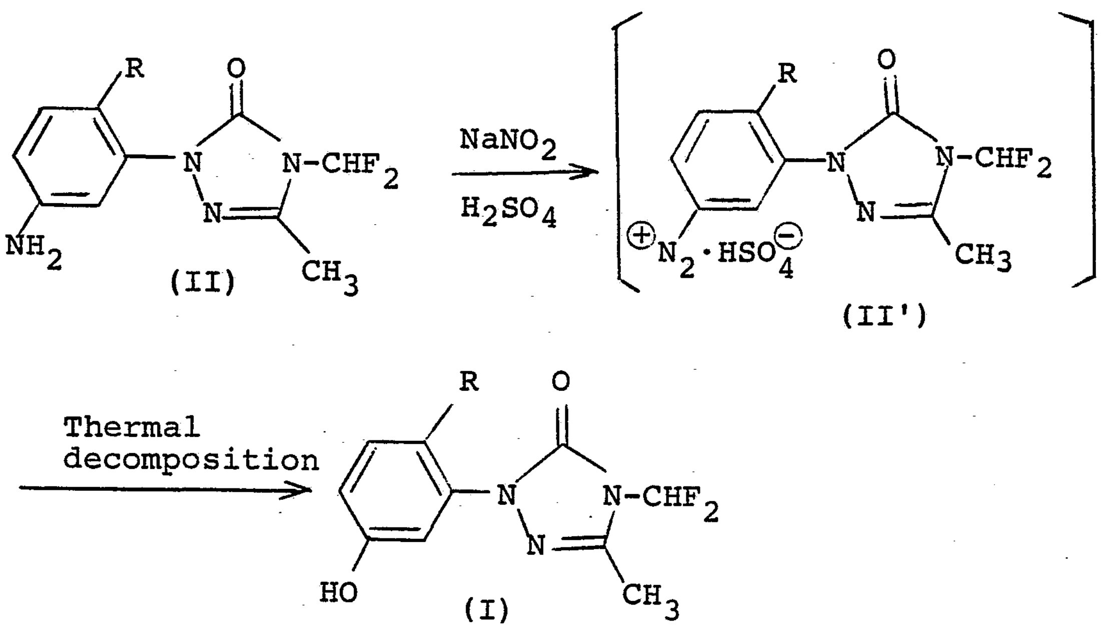


(II) (II') (I)

[wherein R is the same as defined above].

In the above-mentioned diazotization, the reaction may preferably be carried out at a temperature below 5$^{o}$C under a cooling condition. The thermal decomposition may be carried out at a temperature preferably be selected from in the range from a room temperature to 250$^{o}$C. The reaction may be carried out within from 0.5 to 6 hours.

In the diazotization, the amount of sodium nitrite may be selected from in the range from 1.0 to 2.0 moles, and the amount of sulfuric acid may be selected from in the range from 1.5 to 15 moles per one mole of 1-(aminophenyl)-3-methyl-4-difluoromethyl-$\Delta^2$-1,2,4-triazolin-5-one represented by the general formula (II).

After the completion of the decomposition, the crystals formed in the reaction mixture are collected by filtration, and recrystallized or extracted with a solvent, then the extract is dried and the solvent is removed by evaporation to obtain the desired product of 1-(hydroxyphenyl)-3-methyl-4-difluoromethyl-$\Delta^2$-1,2,4-triazolin-5-one represented by the general formula (I).

In the above-mentioned reaction, 1-(aminophenyl)-3-methyl-4-difluoromethyl-$\Delta^2$-1,2,4-triazolin-5-one represented by the general formula (II) used as the starting material may be synthesized, for example by the following method.

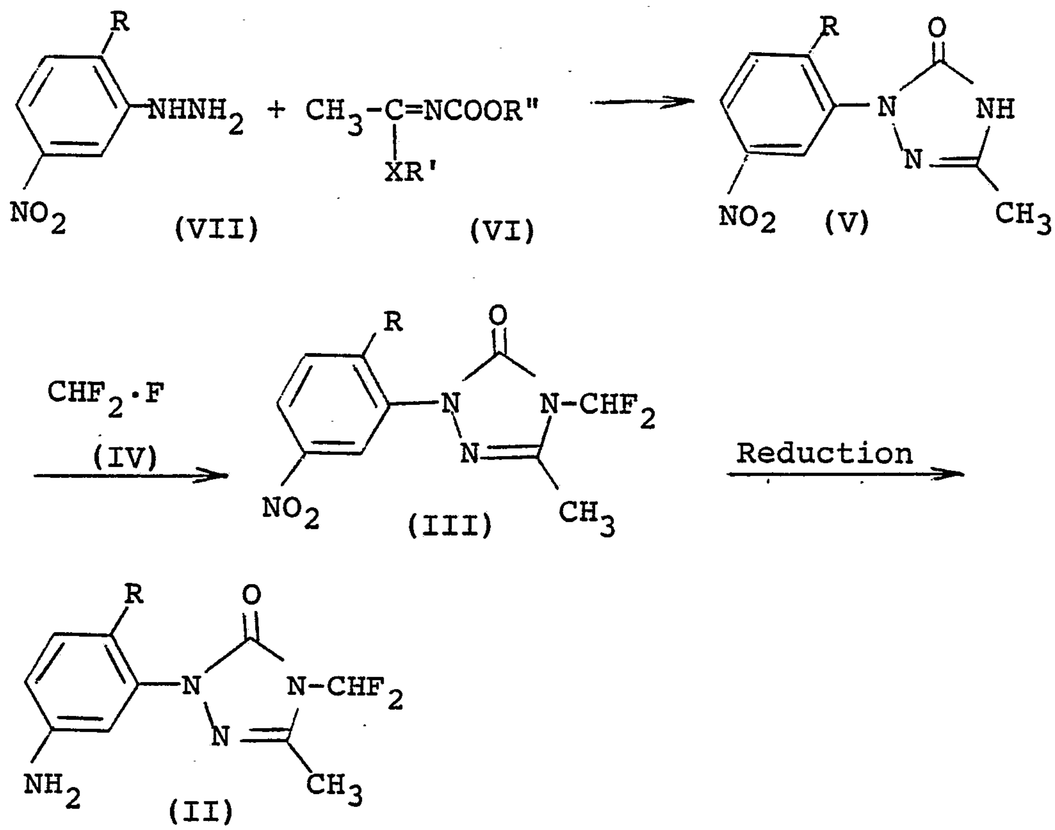


(VII) (VI) (V) (III) (II)

[wherein R is the same as defined above; R' and R" are each a lower alkyl group; X is an oxygen atom or sulfur atom; and Z is a halogen atom].

In the above-mentioned reactions, the ring-closing reaction is carried out by reacting a hydrazine represented by the general formula (VII) with a compound represented by the general formula (VI) in an inert solvent under heating condition to obtain 1-(nitrophenyl)-3-methyl-$\Delta^2$-1,2,4-triazolin-5-one represented by the general formula (V), this product (V) is reacted with a compound represented by the general formula (IV) in an inert solvent to prepare 1-(nitrophenyl)-3-methyl-4-difluoromethyl-$\Delta^2$1,2,4-triazolin-5-one represented by the general formula (III), next this compound (III) is reduced in the presence of a reducing agent to prepare 1-(amino-

phenyl)-3-methyl-4-difluoromethyl-$\Delta^2$-1,2,4-triazolin-5-one.

The present invention will be explained in detail by showing the following Examples, however, the present invention will not be restricted only to these Examples.

Example 1

38 Grams (0.16 mole) of 1-(3-aminophenyl)-3-methyl-4-difluoromethyl-$\Delta^2$-1,2,4-triazolin-5-one was dissolved in 134 ml of 50% (by volume) sulfuric acid, and the solution was cooled to below $0^oC$. Then to this solution was added dropwise 63 ml of an aqueous solution prepared by dissolving 10.9 g (0.16 mole) of sodium nitrite under keeping the reaction temperature at below $0^oC$.

After the dropwise addition of sodium nitrite solution was completed, the reaction mixture was further stirred for 30 minutes, then 350 ml of water was added to the reaction mixture, and further reaction was carried out for 2 hours at $70^oC$. After the reaction was completed, the reaction mixture was cooled, and the crystals formed in the reaction mixture were collected by filtration, and recrystallized from isopropanol to yield 33.9 g of 1-(3-hydroxyphenyl)-3-methyl-4-difluoromethyl-$\Delta^2$-1,2,4-triazolin-5-one.

Melting point: 129 - $132^oC$. Yield 89.3%.

Example 2

0.77 Gram (0.003 mole) of 1-(2-fluoro-5-aminophenyl)-3-methyl-4-difluoromethyl-$\Delta^2$-1,2,4-triazolin-5-one was dissolved in 2.4 ml of 50% (by volume) sulfuric acid, and the solution was cooled at below 0°C. Then to this solution was added dropwise 0.7 g of an aqueous solution prepared by dissolving 0.21 g (0.003 mole) of sodium nitrite by keeping the reaction temperature at below 0°C. After the addition of the sodium nitrite solution was finished, then the reaction mixture was stirred for 30 minutes. The reaction mixture was added dropwise to 4 ml of a 50% sulfuric acid being heated at 160°C. The whole reaction mixture was allowed to stand to cool at a room temperature, and water was added to the reaction mixture. Then the reaction mixture was extracted with ethyl acetate, and dried, then the solvent was removed by evaporation to yield 0.37 g of 1-(2-fluoro-5-hydroxyphenyl)-3-methyl-4-difluoromethyl-$\Delta^2$-1,2,4-triazolin-5-one.
Melting point: 115.5°C. Yield 47.4%.

WHAT IS CLAIMED IS:

1. 1-(Hydroxyphenyl)-3-methyl-4-difluoromethyl-$\Delta^2$-1,2,4-triazolin-5-one derivatives represented by the general formula (I),

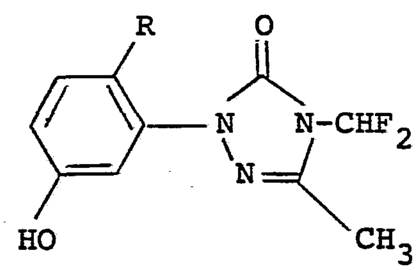

(I)

[Wherein R is a hydrogen atom or a fluorine atom].

2. 1-(3-Hydroxyphenyl)-3-methyl-4-difluoromethyl-$\Delta^2$-1,2,4-triazolin-5-one.

3. 1-(2-Fluoro-5-hydroxyphenyl)-3-methyl-4-difluoromethyl-$\Delta^2$-1,2,4-triazolin-5-one.

4. A method of preparing compounds of the general formula (I), as defined in Claim 1, which comprises diazotizing a compound of the general formula (II),

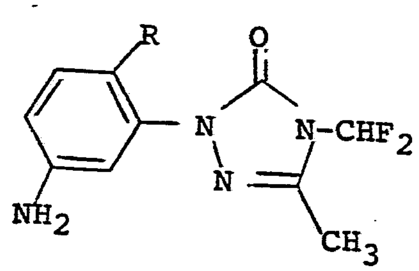

(II)

wherein R is as defined in Claim 1, and thermally decomposing the diazotized product.

5. Use of compounds of the general formula (I), as defined in Claim 1, in the preparation of agricultural chemicals.

6. Use of compounds of the general formula (I), as defined in Claim 1, in the preparation of herbicides.

0193660

European Patent Office

# EUROPEAN SEARCH REPORT

Application number

EP 85 30 1443

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | GB-A-2 090 250 (NIHON NOHYAKU CO. LTD.) * claims 1-3,17-19; table 1, compound no. 12 * | 1-3,5, 6 | C 07 D 249/12<br>A 01 N 43/64 |
| A | PATENT ABSTRACTS OF JAPAN, vol. 7, no. 277 (C-199)[1422], 9th December 1983; & JP - A - 58 157 771 (NIPPON NOYAKU K.K.) 19-09-1983 | 1,5,6 | |
| A | PATENT ABSTRACTS OF JAPAN, vol. 8, no. 71 (C-217)[1508], 3rd April 1984; & JP - A - 58 225 070 (NIPPON NOYAKU K.K.) 27-12-1983 | 1,5,6 | |
| A | EP-A-0 055 105 (SUMITOMO CHEMICAL CO., LTD.) * claims 1,7,8 * | 1,5,6 | TECHNICAL FIELDS SEARCHED (Int. Cl.4)<br>C 07 D 249/00 |

The present search report has been drawn up for all claims

| Place of search | Date of completion of the search | Examiner |
|---|---|---|
| BERLIN | 14-10-1985 | HASS C V F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82